# EUROPEAN PATENT SPECIFICATION

(11) **EP 2 339 323 B1**
(45) Date of publication and mention of the grant of the patent: **08.05.2019**
(21) Application number: 09466006.5
(22) Date of filing: 05.06.2009
(51) Int. Cl.: G01N 11/00, G01N 11/06, G01F 3/28

(54) **The method of simulation of kinetics movement of bulk solid particles and facilities to carry out the method**
Verfahren zur Simulation der kinetischen Bewegung von festen Schüttpartikeln und Anlagen zur Durchführung des Verfahrens
Procédé de simulation de mouvement cinétique de particules solides en vrac et installations permettant d'effectuer le procédé

(43) Date of publication of application: 29.06.2011
(73) Proprietor: Vysoka Skola Banska-Technicka Univerzita, 708 33 Ostrava - Poruba (CZ)
(72) Inventor: Zegzulka, Jiri, 747 14 Ludgerovice (CZ); Bortlik, Petr, 747 23 Bolatice (CZ); Dokoupil, Otakar, 751 21 Prosenice (CZ); Brazda, Robert, 708 00 Ostrava - Poruba (CZ); Necas, Jan, 720 00 Ostrava - Hrabova (CZ)
(74) Representative: Kendereski, Dusan

(56) References cited:
- EP-A1- 1 977 737
- GB-A- 977 282
- GB-A- 1 547 692
- US-A- 2 045 193
- US-A- 4 716 768
- US-A- 5 165 291
- US-A1- 2009 078 029
- US-B1- 6 367 336
- US-B1- 6 472 615
- ERIC I. CORWIN: "granular flow in a rapidly rotated system with fixed walls", PHYSICAL REVIEW, vol. 77, no. 031308, 19 March 2008 (2008-03-19), pages 1-8, XP002643377,

## Description

### Technical Field

The invention relates to identifying the properties of bulk solids, and solves the implementation of control or simulation experiments for the verification of engineering works and the research activities in the field of application processes connected with bulk solids handling, in particular the simulation of the course of pressures, the size of flow profiles, the simulation of flow and velocity fields, circumfluence of the passive elements, corners, barriers, etc., among other things the structure of the composition of bulk solids, including the structure of particle nanoaggregates.

### Description of Prior Art

It is known that movement of the bulk solid materials results in the individual movement of particles. The movement of each particle is affected by both the properties of the particles and the properties of the surroundings by which a particle is surrounded. If the bulk solid consists of particles of different properties, such as different sizes, different densities, different shapes, or if the particles are differently shaped or have a different rough surface, the clustering of particles, which were originally distributed in the bulk solid matter evenly occurs when moving the bulk solid material. This leads to a preferential clustering of certain particles in specific locations, such as clustering of larger or heavier particles at the bottom of a silo, at the bottom of a chute, or in places where the changes of movement direction occur, etc. This phenomenon is particularly undesirable if the different properties of particles are caused by their different chemical composition. Subsequently, originally a relatively homogeneous mixture becomes, after the movement, clearly inhomogeneous, which when dosing the bulk solid mixture in smaller amounts will cause undesirable variations in the chemical composition of individual doses. To eliminate an influence of such phenomena as much as possible, it is necessary to design an operational device for the application sphere so as to eliminate the undesirable phenomena.

There are two groups of methods known for monitoring the behaviour of bulk solid materials in movement. The first group of methods focuses on the measurement of phenomena taking place in real devices using physical methods. This concerns the measurement of pressures, forces, kinematics and dynamics of the particles' movement, shocks, interreactions with the construction, etc. To simulate the behaviour of bulk solid materials in real devices it is also possible to use the model bulk solid material. The disadvantage of this solution is that the operational deficiencies of the device are discovered only after its manufacture, which requires that the operational device can be reconstructed only based on practical results, which is associated with high costs. The second group of methods uses for the simulations the processes of mathematical modeling of movement and behaviour of particles in the simulation devices that are compared to the real devices created in a reduced or enlarged scale. The disadvantage of such simulation devices and the methods used is that the processes occurring in the bulk solids are modeled against the vertical axis in the actual position as in the operational device or in the actual position of process, transport or storage devices, and do not track the effects of deviations in the structural design on the behaviour of bulk solid materials. For example, there are known simulation methods applied to the fixed, vertically oriented simulation devices that do not allow tilting from the tilting axes, on which the studies of bulk solid properties in the process simulations, which in practice take place in bulk solid materials, are conducted. The simulation studies are carried out on simulation devices, either with simulation materials or directly with actual materials, which are to be used in an operational device. The results identified through the simulation method are then transmitted using different criteria to an operational device. The simulation materials in order to be used for the simulation, carried out by the simulation device, must be inert, and also isotropic on a long-term basis, and stable. The simulation materials such as plastic or metal pellets, cylinders, prisms, and chips of crushed limestone are used. When using the simulation materials on the operational device, the properties of the actual material, which the bulk solid material in the simulation experiment did not show, may be significantly displayed leading to the need for subsequent adjustments to an operational device. When using actual material in the simulation device, its properties may change during the simulations due to degradation, segregation, long-term contact with air in oxidizing or hygroscopic materials, or the properties can change as a result of repeated experiments, for example the larger particles can be crushed due to the long-term pouring, so that the bulk solid behaves, during repeated simulations, differently than it would behave in an operational device. The common known disadvantage of simulation methods is a questionable credibility of information transfer from the simulation device into the operational device, which is dependent on the method used and the experience of the experimenter as well as the dependence on the existence of a simulation device, which is expensive, while the option of conducting experiments by outsourced contractors is at risk of disclosure of information.

Mathematical modeling of the behaviour of bulk solid materials using methods such as the method of finite elements or discrete elements is also known. The disadvantage of mathematical modeling is that these methods are not yet processed at a level such that a satisfactory operational device can be proposed with better risk profile.

A method of quantitative evaluation of flow properties of pharmaceutical powder which flows over a time interval on a free surface of a vibrating inclined plane and which falls after passing a lower edge of that inclined plane on the weighting means where it is weighted, is disclosed in the patent application EP 1977737 A1. The flowing powder material is in a kinetic state and not under the stress transition state. The powder matearial moves on the free surface without restriction of a layer thicknes by any walls, so it is not possible to observe cross section layers. Except vibrations the inclined plane is static and it does not allow to perform dynamic changes, like rotation or change in any tilting angle.

Simillarly in the document US2009/0078029 a device consisting of a vertical pipe attached to a vibrator and having a circular crosssection outlet opening at its bottom is described. Powder material that passes through the outlet opening over the selected time period is also weighted. Due to round shape of the pipe it is not possible to observe any layer of passed material.

Also, the patent document US 4716768 discloses a silo full of granular material where after the bottom cover is openned the material flows over a selected time period through the bottom opening and it is weighted.

In the patent document GB 977282 A, there is an hourglass described, which consists of a neck which connects two glass bulbs that are attached to the frame with two joints enabling clockwise or counterclockwise rotation in one plane arround one stable axis of rotation, but not allowing rotation in arbitrary direction or inclination of of that plane. The hourglass is used to measure constant period of time in which all sand passes the neck. Due to round shape of bulbs it is not possible to observe behaviour in layers. The over all picture observed in those bulbs is distorted. Moreover the neck is not adapted for testing, thus it does not enable to change shapes or dimensions of a slot for testing. The shape of hourglass is constant and does not allow to simulate crossections layers of real devices.

### Disclosure of Invention

The disadvantages stated are addressed by the method and testing device for simulation of the movement kinetics of bulk solid particles from the invention.

The principle of the method is defined in claim 1, and its dependents.

The principle of the testing device is defined in claim 3 and its dependents.

The advantage of the method from the invention is the possibility of searching critical areas in the micro-processes running in the bulk solids on individual particles as well as in the ongoing macro-processes on particle aggregates, and when seeking solution to the boundary conditions, in the contact of the device wall with the bulk solid material, and furthermore, the possibility to optimize the requirements for mechanical and physical properties of the bulk solid materials to meet these stabilization processes and their required speeds and properties. A major advantage is the possibility to optimize the selection of contact materials and research on the optimal design shape of the engineering work. An advantage of the method used in the invention is a further its complexity, the possibility of setting the methodology to address the issues of segregation, degradation, flow continuity, arching, dynamic properties of the flow and many other phenomena. Another advantage is the large range of simulations with regard to the needs of industry, such as the pressure simulation and its usage applications, peak pressure, simulation of dynamic properties of the flow, pressing, and movement of material during pressing. Another advantage is the possibility to optimize the positions, shapes and construction materials used on the actual testing device based on the simulations carried out on the simulation device in relation to actual properties of the bulk solid material, with which the operational device will manipulate.

The advantage of the testing device in the invention is that the properties of bulk solid material show proportionally during simulation, in a fundamental physical form, thus allowing mapping of the flow. When using a simulation material, another advantage is independence from industry, in which the results are to be used and the type of bulk solid material is actually used.

Generally speaking, the benefits of the simulations according to the invention include obtaining information on the behaviour of the particulate matter in the process of its flow through the opening for the micro and macro research of the particulate matter system, in the flow through the cone, such as through the bottom of a silo. Another benefit is the simulation of the formation of different structures of the bulk solid particle arrangements under different conditions, studies of the effect of changing properties of the matter, especially particle shape, granulometry, bond simulations, etc. It is also possible to study the boundary conditions determined by the shape of the space in which the movement takes place, both in terms of its shape, and in terms of contact materials, which limit this subspace. The simulation can also be carried out to obtain information on the movement of the matter in different environments with different gravity or acceleration, etc.

### Description of the Drawings

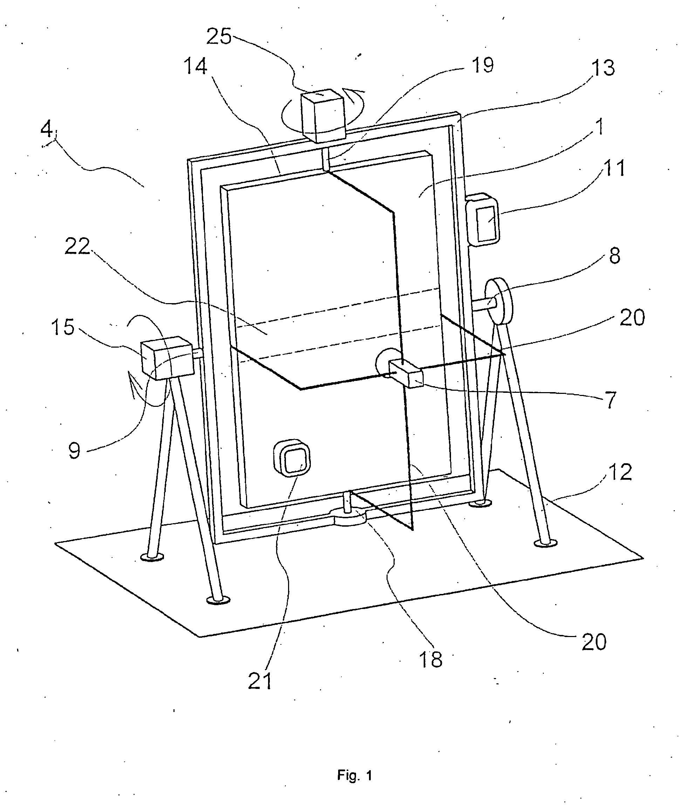

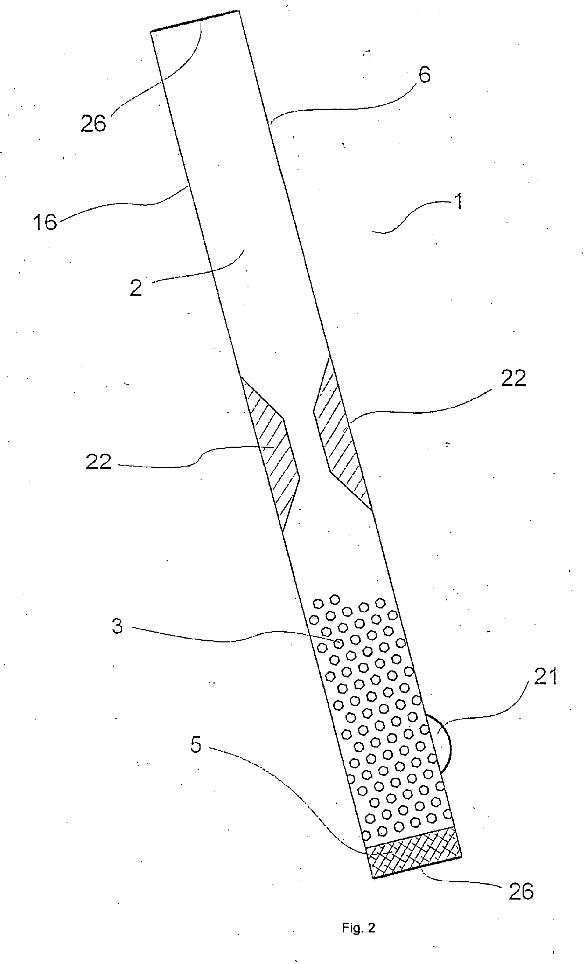

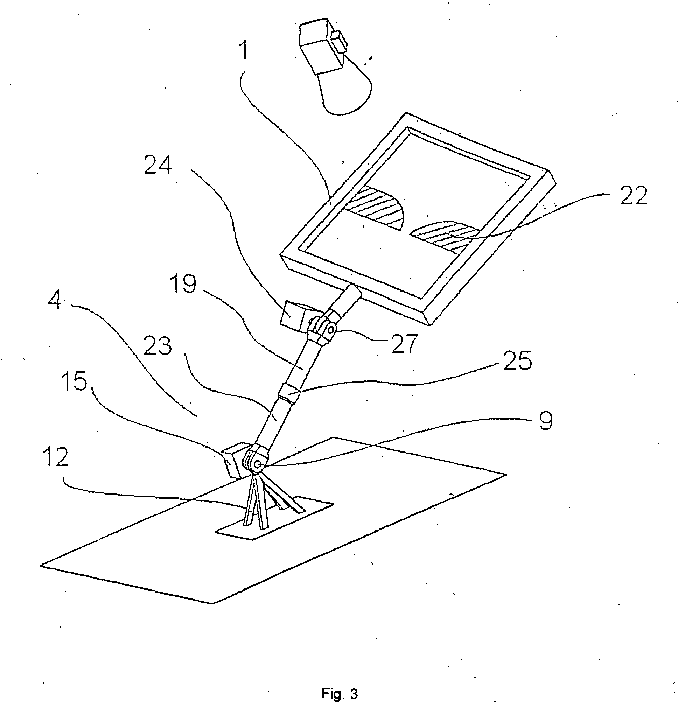

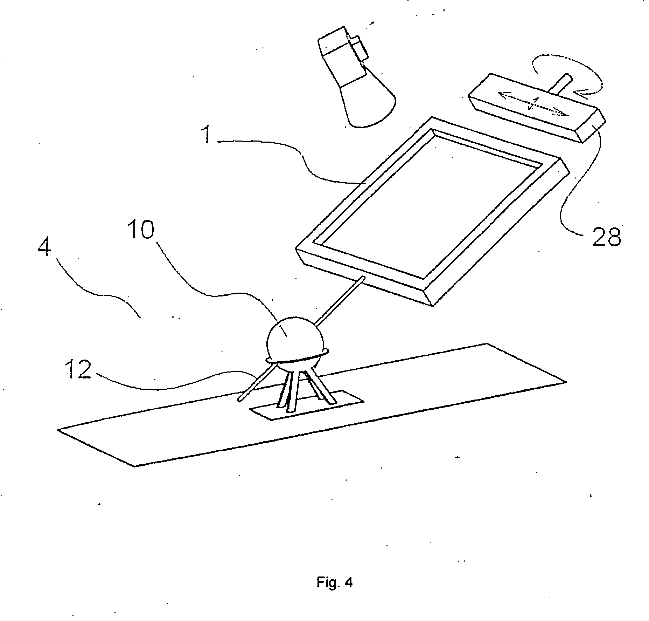

Figure 1 schematically shows the simulation device in accordance with Example 1, Figure 2 shows a cross-section of the model chamber in accordance with Example 1. Figure 3 shows the simulation device from Example 2, and Figure 4 shows the simulation device in accordance with Example 3.

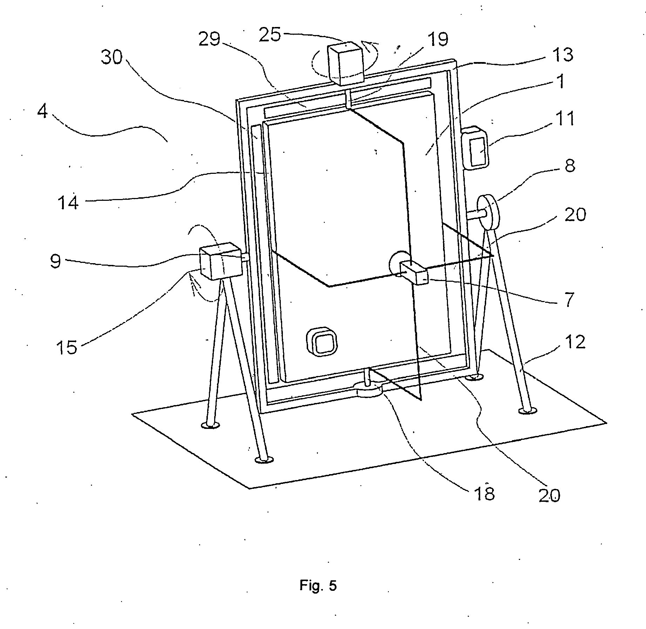

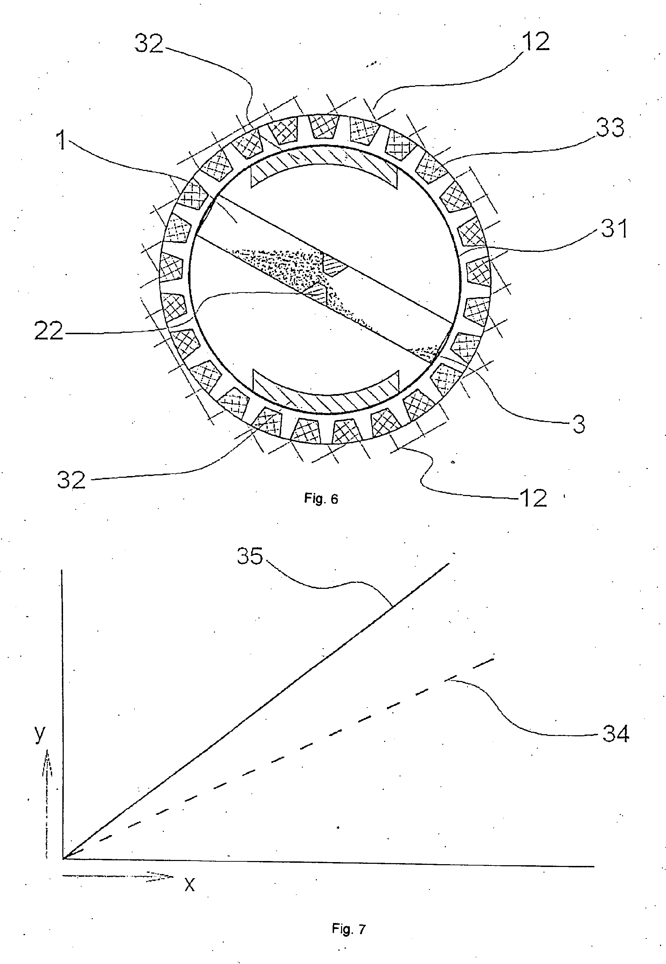

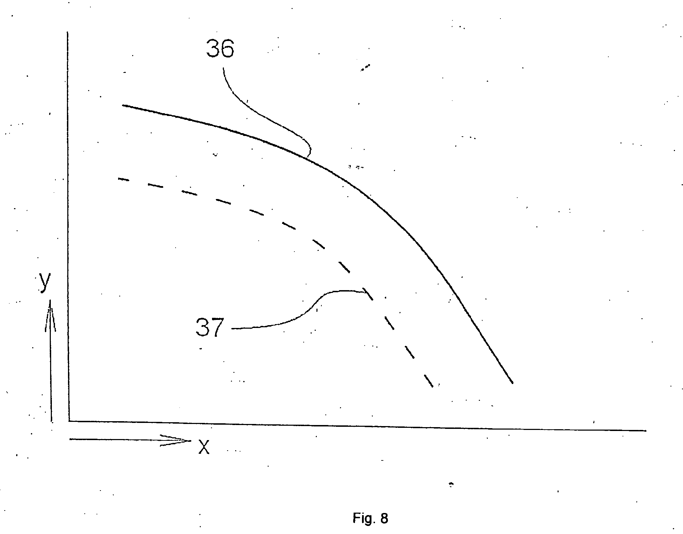

Figure 5 shows the same in accordance with Example 4, and Figure 6 shows the same in accordance with Example 5. Figure 7 shows the dependence of the height of static and dynamic arch above the outlet opening as a function of the size of the outlet opening, at a constant angle of internal friction for Example 1, and Figure 8 represents the dependence of the height of static and dynamic arch above the outlet opening of the constant size as a function of the variable size of the angle of internal friction for Example 2.

### Examples of Embodiments

### Example 1

A testing device for simulation of the movement kinetics of bulk solid particles according to Example 1 consists of a positioning device 4, which consists of a supporting structure 12, to which a main rotating frame 13 is rotationally adjusted through the first pin 8 and the first shaft 9. In the main rotating frame 13 an auxiliary rotating frame 14 is rotationally adjusted through the second pin 18 and the second shaft 19. The first shaft 9 represents a shaft of the first drive 15 and the second shaft 19 represents a shaft of the second drive 25. The axes of rotation of both shafts 9, 19 are perpendicular to each other. The main rotating frame 13 is connected to the first vibrator 11. The testing device also includes a model box 1, which is dismountably connected with the auxiliary rotating frame 14. The model box 1 consists of a front wall 6, a rear wall 16, which are both transparent and parallel to each other, and side walls 26, whose width determines the distance between the front wall 6 and the rear wall 16. The front wall 6 of the model chamber 2 , is fitted with the second vibrator 21. Sensors 5 of the bulk solid are located on the inner surfaces of side walls 26, in this case, the pressure sensors. The walls 6, 16, 26 of the model box 1 define the model chamber 2, in which the bulk solid 3 is located, consisting of spherical particles of the model material. The model chamber 2 profile is narrowed in the middle part by the barriers 22, defining the hole-shaped opening of a rectangular cross-section. The auxiliary rotating frame 14 is further fitted with an observation unit 7, a video camera, using struts 20. The positioning device 4 allows a tilt of the model box 1 in any direction and/or rotation of the model box 1 around axis oriented in any direction clockwise and counterclockwise, with the bulk solid 3_moving in the model chamber 2, and its movement is affected by the bottleneck profile due to the barriers 22. Dimensions of the model box 1, including the shape and size of the barriers 22, are defined with regard to the objective of the research.

The method according to Example 1 is performed so that the examined bulk solid 3 is inserted in the horizontally oriented model box 1, in this case the balls, which have a constant angle of internal friction. The model box 1 subsequently tilts by increasing the angle of tilt in a direction which is perpendicular to the longitudinal axis of the opening hole defined by the barriers 22 until reaching an angle of tilt 34 °, when the power balance is reached, and as its result the movement of the bulk solid 3 in the model box 1 is even. The angle of tilt of the model box 1 is subsequently continuously changing in the range of ± 5 °, with the aim of creating a stable position of both the static and dynamic arch. Then the geometric values of dynamic and static arch position are deducted. The measurement described above is carried out repeatedly for various sizes of opening holes, thus for the various width of the slot between the barriers 22. Measured values are transcribed to the dependency graph of the height of static and dynamic arch above the opening hole, see y-axis in Figure 7, on the size of the opening hole, see x-axis in Figure 7, for a constant angle of internal friction. Furthermore, the centre of the coordinate system and experimentally determined heights of static and dynamic arch are fitted with the lines, and thus to obtain the course 34 of the static arch height depending on the width of the slot, and the course 35 of the dynamic arch height depending on the width of the slot, which is valid provided that the angle of internal friction is a constant. The height of both the static and dynamic arches for other slot widths, for which the measurement was not conducted, are deduced from this graph. Geometrical parameters of the static and dynamic arch depend on geometrical dimensions of engineering works and thus on the scale of the model, and therefore the values for actual work are determined from the experimental values by the conversion.

### Example 2

A testing device according to Example 2 differs from the device described in Example 1 in that the barriers 22, which are located inside the model box 1 define the length of the slot-shaped opening hole, whose width is determined by the mutual distance of the walls 6,16 of the model box 1, and in that the positioning device 4, which connects the model box 1 with the base 12, is composed of an adapter 23, which can be tilted using the first drive 15 against the base 12, of the second shaft 19, which can be coaxially rotated using the second drive 25 in the adapter 23, and of the third drive 24, through which it is possible to tilt the model box 1 toward the second axis of the shaft 19. The first drive 15 is connected to the base 12 and is connected using the first shaft 9 to the first end of the adapter 23, while the first axis of the shaft 9 is perpendicular to the axis of the adapter 23. The other end of the adapter 23 is fitted with a second drive 25 accompanied by the second shaft 19, the axes of the second adapter 23 and the second shaft 19 are identical. At the end of the second shaft 19, which is located in the opposite site to the adapter 23, there is the third drive 24, connected through the third shaft 27 with the model box 1, while the axis of the third shaft 27 is perpendicular to the axis of the second shaft 19.

Cement, representing the examined bulk solid material 3 is inserted in the model box 1 according to Example 2. For cement, the situations can be modeled where the bulk solid material particles do not have a constant angle of internal friction, because the angle of internal friction in the cement depends not only on the height of the layer, even if the height of the layer is simulated by weights, but also, for example, on the absorbed moisture content, as cement is hygroscopic and changes its properties in a humid environment over time. The model box 1 is filled with cement in a horizontal position and is gradually tilted from this position by increasing the tilt angle in the first direction, which is perpendicular to the longitudinal axis of the opening hole defined by barriers 22, up to an angle 34 °, at which a uniform movement of the bulk solid occurs in the model box 1 for the first direction, where, furthermore, the tilt angle of the model box 1 continuously changes in the first direction in the range of ± 5 ° in order to create a stable position of both, the static and dynamic arch. Then, model box 1 is gradually tilted by increasing the tilt angle in the second direction, which is perpendicular to the first direction, up to an angle 28 °, where there is the force balance for the combined effects of tilt in the first and second tilt directions, in which the transition of the plane stress state into the spatial stress state, which results in changing the position of static and dynamic arch in the model box 1 due to inconstant angle of internal friction. The tilt angle of the model box 1 also continuously changes in the second tilt direction in the range of ± 4 °, in order to stabilize force balance. The experiment noted is repeated for different heights of the bulk solid above the slot, leading to changes in the value of the angle of internal friction. Greater heights can be simulated using the load placed above the bulk solid material. The measured values for one particular slot are plotted in the graph in Figure 8, where the x-axis represents the angle of internal friction, and the y-axis the height of the static or dynamic arch. The measured values recorded in the graph are connected using the extrapolation curves to obtain the dependence of the static arch height 36 on the angle of internal friction and the dependence of the dynamic arch height 37 on the angle of internal friction. From the graphs for each size of outlet opening height, the heights of both the static and dynamic arch, such as dependence on the angle of internal friction for the specific size of an outlet opening can be determined. By combining the graphs for different sizes of outlet openings, the height of a static or dynamic arch for the specific size of outlet opening and the specific size of internal friction can be seen. Geometrical parameters of both the static and dynamic arch depend on the geometrical dimensions of the engineering works and thus on the model scale, and also on the angle of internal friction and its changes.

### Example 3

A testing device for simulation of the movement kinetics of bulk solid particles according to Example 3 differs from Example 2 in that the positioning device 4, by which a model box 1 is connected to the base 12, is formed by the joint 10, which allows the tilt at a spatial angle. And furthermore, the device differs by the fact that there are no other barriers inside the model box 1. A permanent magnet 28 is placed facing the opposite side of the upper edge of the model box 1, which can be moved in space and using the described mechanism rotated, while the model chamber 2 rotates or tilts around an axis passing through the centre of the joint 10.

The method according to Example 3 differs from the method described in Example 1 in that the bulk solid is particles of dry sand that are moistened by a defined amount of water prior to insertion into model box 1, thereby modeling the links between particles of sand. Repeated measurements of sand with varying amounts of water poured can be use to monitor the dependence of the static and dynamic arch height on the bond strength between particles.

### Example 4

Example 4 differs from Example 1 in that the device is designed to monitor the behavior of ferromagnetic powder. A model box 1 is therefore supplemented by permanent magnets 29, 30. The first permanent magnet 29 is located along the upper edge of the model box 1, while the second permanent magnet 30 is located along the left side wall 26.

### Example 5

A testing device for simulation of the movement kinetics of bulk solid particles according to Example 5 differs from Example 1 in that model chamber 2 is with the base 12 bonded by force. A model box 1 is located in the spherical body 31, which rotates on any axis, depending on the direction and intensity of the magnetic field between the first, exemplary permanent magnets 32 located inside the spherical body 31, and the second magnets 33, in an examplary manner the electromagnets, which are connected with the base 12, whose coils surround the spherical body 31. The bulk solid according to this example is an electrostatically charged laundry detergent. By changing the size of current flow direction, passing through the coils of the second magnets 33, the tilt of the model chamber 2 or its changes can be adjusted.

### Industrial Applicability

The invention can be used to support pre-project preparation, project processing and optimization of structural design. The testing device from the invention can be used for research and development of new engineering solutions for process equipment, conveyors, storage facilities, laboratory equipment, etc., to optimize the engineering works seeking the optimal contact design materials and optimal shapes of structures designed to ensure the continuity and desired speed of the processes. Tendency towards segregation, degradation and other negative processes are detected using simulation of conditions and states on the testing device from the invention.

By implementation of the method from the invention the information regarding the behaviour of the bulk solid material in the process of its flow through the opening in the micro and macro scale, such as examining the behaviour of bulk solid when flowing through a cone or the bottom of a silo, can be obtained. Also, it is possible to simulate the formation of different structures of the bulk solid particles arrangement under various conditions, examine the influence of changeable properties of the bulk solid material, such as a particle shape, granulometry, bond simulation, and furthermore, the boundary conditions given by the shape of the space, in which the movement takes place, both in terms of its shape or aspects of contact materials that define this space. The simulations can be further carried out in order to obtain information on the movement of the bulk solids in different gravity environments, environments with different acceleration, etc.

## Claims

1. A method of determination of a dynamic arch height of bulk solid (3), **characterized in that** after insertion of the bulk solid (3) in a horizontally oriented model box (1) with a parallel transparent front wall (6), a rear wall (16) and barriers (22) defining a slot between the barriers (22) a tilt angle between the model box (1) and the horizontal plane is increased in at least one direction by a positioning device until a uniform movement of the bulk solid (3) occurs in the model box (1) in the first direction and then the tilt angle is subsequently continuously changing in the first direction in the range of ± 5° for creating a stable position of both the static and dynamic arch, while the geometric values of dynamic arch and its position are deducted with use of a video camera, while the measurement is repeated with various values of one of the following chosen parameters: various width of the slot between barriers (22) in the model box (1), various content of added water to bulk solid (3) changing bond strength between particles, or using different heights of the bulk solid (3) above the slot; measured values of the dynamic arch height are then transcribed to a graph of dynamic height in dependency on the chosen parameter.

2. A method of determination of a dynamic arch height of bulk solid (3) according to claim 1, **characterized in that** the tilting and/or rotating is accompanied by vibration and/or by the effects of a magnetic force.

3. A testing device for determination of a dynamic arch height of bulk solid (3) according to claim 1 or 2 containing a model box (1) containing a model chamber (2) for bulk solid (3) insertion, whereas the model chamber (2) is surrounded by walls (6, 16, 26) and includes barriers (22) defining a slot between the barriers (22), where the walls are a front wall (6), a rear wall (16), which are at least partly transparent and parallel to each other, and side walls (26), and comprising a positioning device (4) that is connectd to the model box (1) and is comprising a base (12), the positioning device (4) is arranged for rotation and/or tilting of the model box (1) around at least two not parallel axis; the testing device further contains a video camera (7) for observation of the bulk solid via the transparent part of the walls.

4. A testing device according to claim 3, **characterized in that** the model box (1) is further supplemented by permanent magnets (29, 30), a first permanent magnet (29) is located along the upper edge of the model box (1), a second permanent magnet (30) is located along the side wall (26).

5. A testing device according to claim 3 or 4, **characterized in that** the positioning device (4) consists of a base (12), to which a main rotating frame (13) is rotationally adjusted through a first pin (8) and a first shaft (9) with a first drive (15), where an auxiliary rotating frame (14) is rotationally adjusted in the main rotating frame (13) through a second pin (18) and a second shaft (19) with a second drive (25), whereas the axes of rotation of both shafts (9, 19) are perpendicular to each other; the model box (1) is connected with the auxiliary rotating frame (14) and the video camera is fitted on the auxiliary rotating frame (14), where the first pin (8) and the first shaft (9) are connected to the base (12).

6. A testing device according to claim 5, **characterized in that** the main rotating frame (13) is further connected to a first vibrator (11) and the front wall (6) of the model chamber (2) is fitted with a second vibrator (21).

7. A testing device according to claim 3 or 4, **characterized in that** the positioning device (4) includes a first drive (15) connected with the base (12), a first drive (15) with a first shaft (9) that is further connected to an adapter (23), a second drive (25) that is a connection between adapter (23) and the second shaft (19), the second shaft (19) connected via a third drive (24) to the third shaft (27) further connected to the model box (1), while the first axis of the shaft (9) is perpendicular to the axis of the adapter (23) and the axis of the second adapter (23) and the second shaft (19) are identical, while the axis of the third shaft (27) is perpendicular to the axis of the second shaft (19).

8. A testing device according to claim 3 or 4, **characterized in that** the positioning device (4) contains a joint 10, for tilting/rotation of a model box (1) at a spatial angle and a permanent magnet (28) for rotation the model box (1) that is placed facing the opposite side of the upper edge of the model box (1), which can be moved in space.

9. A testing device according to claim 3 or 4, **characterized in that** the positioning device includes a spherical body (31) inside which the model box (1) is located, which rotates around any rotation/tilting axis, depending on the direction and intensity of the magnetic field between the first permanent magnets (32) located inside the spherical body (31), and the second magnets (33) connected with the base (12) and surrounding the spherical body (31).

10. A testing device according to any claim from 3 to 9 **characterized in that** the model box (1) further contains a pressure sensor (5) of the bulk solid (3) located on the inner surfaces of side walls (26) and/or an audio signal sensor and/or an ultrasonic sensor.

## Patentansprüche

1. Ein Verfahren zur Bestimmung einer dynamischen Bogenhöhe von Schüttgut (3), **dadurch gekennzeichnet, dass** nach dem Einsetzen des Schüttgutes (3) in einer horizontal ausgerichteten Modellbox (1) mit einer parallelen transparenten Vorderwand (6), einer Rückwand (16) und Barrieren (22), die einen Schlitz zwischen den Barrieren (22) definieren, wobei ein Neigungswinkel zwischen der Modellbox (1) und die Horizontalebene durch eine Positioniereinrichtung in mindestens einer Richtung soweit vergrößert wird, bis eine gleichmäßige Bewegung des Schüttgutes (3) in der Modellbox (1) erfolgt in der ersten Richtung und anschließend der Kippwinkel kontinuierlich in der ersten Richtung im Bereich von ± 5° verändert wird, um eine stabile Lage sowohl des statischen als auch des dynamischen Bogens zu erzeugen, während die geometrischen Werte des dynamischen Bogens und dessen Position unter Verwendung einer Videokamera abgezogen werden, während die Messung mit verschiedenen Werten eines der folgenden gewählten Parameter wiederholt wird: unterschiedliche Breite des Schlitzes zwischen Barrieren (22) in der Modellbox (1), verschiedene Inhalte von zugesetztem Wasser zum Schüttgut (3), die der Klebkraft zwischen Partikeln verändern, oder unterschiedlichen Höhe des Schüttgutes (3) oberhalb des Schlitzes; dann werden Messwerte der dynamischen Bogenhöhe transkribiert einem Graphen der dynamischen Höhe in Abhängigkeit von dem gewählten Parameter.

2. Das Verfahren zur Bestimmung einer dynamischen Bogenhöhe von Schüttgut (3) nach Anspruch 1, **dadurch gekennzeichnet, dass** das Kippen und/oder das Drehen mit einer Vibration und/oder durch die Einwirkung einer magnetischen Kraft einhergeht.

3. Eine Prüfeinrichtung zur Ermittlung einer dynamischen Bogenhöhe von Schüttgut (3) nach Anspruch 1 oder 2, umfassend eine Modellbox (1), die eine Modellkammer (2) zum Einschieben des Schüttgutes (3) aufweist, während die Modellkammer (2) von Wänden (6, 16, 26) umgeben ist und Barrieren (22) umfasst, die einen Schlitz zwischen den Barrieren (22) definieren, wobei die Wände eine Vorderwand (6), eine Rückwand (16), die zumindest teilweise transparent und parallel zueinander sind, und Seitenwänden (26), und eine Positioniereinrichtung (4) umfassen, die mit der Modellbox (1) verbunden ist und einen Boden (12) aufweist, wobei die Positioniereinrichtung (4) zur Drehung und/oder zur Verkippung der Modellbox angeordnet ist (1) um mindestens zwei nicht parallele Achsen; die Prüfvorrichtung weist ferner eine Videokamera (7) auf zur Beobachtung des Schüttgutes über den transparenten Teil der Wände.

4. Die Prüfvorrichtung nach Anspruch 3, **dadurch gekennzeichnet, dass** die Modellbox (1) weiterhin durch Permanentmagnete (29, 30) ergänzt ist, wobei ein erster Permanentmagnet (29) entlang der Oberkante der Modellbox (1) angeordnet ist, ein zweiter Permanentmagnet (30) entlang der Seitenwand (26) angeordnet ist.

5. Die Prüfeinrichtung nach Anspruch 3 oder 4, **dadurch gekennzeichnet, dass** die Positioniereinrichtung (4) aus einem Boden (12) besteht, an dem ein Hauptdrehrahmen (13) durch einen ersten Bolzen (8) und eine erste Welle (9) mit einem ersten Antrieb (15) drehverstellt ist, wobei ein Hilfsdrehrahmen (14) in dem Hauptdrehrahmen (13) durch einen zweiten Bolzen (18) und eine zweite Welle (19) mit einem zweiten Antrieb (25) drehverstellt ist, wobei die Drehachsen beider Wellen (9, 19) lotrecht zueinander stehen; die Modellbox (1) mit dem Hilfsdrehrahmen (14) und die Videokamera an dem Hilfsdrehrahmen (14) angebracht ist, wobei der erste Bolzen (8) und die erste Welle (9) mit dem Boden (12) verbunden sind.

6. Die Prüfvorrichtung nach Anspruch 5, **dadurch gekennzeichnet, dass** der Hauptdrehrahmen (13) ferner mit einem ersten Vibrator verbunden ist (11) und die Vorderwand (6) der Modellkammer (2) mit einem zweiten Vibrator (21) ausgestattet ist.

7. Die Prüfvorrichtung nach Anspruch 3 oder 4, **dadurch gekennzeichnet, dass** die Positioniervorrichtung (4) einen mit dem Boden (12) verbundenen ersten Antrieb (15), einen ersten Antrieb (15) mit einer ersten Welle (9) aufweist, die ferner mit einem Adapter (23) verbunden ist, einen zweiten Antrieb (25), die eine Verbindung zwischen dem Adapter (23) und der zweiten Welle (19) ist, die zweite Welle (19), die über einen dritten Antrieb (24) mit der dritten Welle (27) verbunden ist, die weiter mit der Modellbox verbunden ist (1), während die erste Achse der Welle (9) lotrecht zur Achse des Adapters (23) ist und die Achsen des zweiten Adapters (23) und der zweiten Welle (19) identisch sind, während die Achse der dritten Welle (27) lotrecht zur Achse der zweiten Welle (19) ist.

8. Die Prüfvorrichtung nach Anspruch 3 oder 4, **dadurch gekennzeichnet, dass** die Positioniereinrichtung (4) ein Gelenk (10) zum Kippen/Drehen einer Modellbox (1) um einen Raumwinkel und einen Permanentmagneten (28) zum Drehen der Modellbox (1) aufweist, die der gegenüberliegenden Seite der Oberkante der im Raum verfahrbaren Modellbox (1) zugewandt angeordnet ist.

9. Die Prüfvorrichtung nach Anspruch 3 oder 4, **dadurch gekennzeichnet, dass** die Positioniereinrichtung einen Kugelkörper (31) umfasst, innerhalb dessen sich die Modellbox (1) befindet, der sich um eine beliebige Dreh-/Kippachse dreht, in Abhängigkeit von der Richtung und Stärke des Magnetfeldes zwischen den im Inneren des Kugelkörpers (31) befindlichen ersten Permanentmagneten (32) und den mit dem Boden (12) verbundenen und den Kugelkörper (31) umgebenden zweiten Magneten (33).

10. Die Prüfvorrichtung nach einem der Ansprüche 3 bis 9, **dadurch gekennzeichnet, dass** die Modellbox (1) ferner einen Drucksensor (5) des auf den Innenflächen von Seitenwänden (26) befindlichen Schüttgutes (3) und/oder einen Audiosignalsensor und/oder einen Ultraschallsensor aufweist.

## Revendications

1. Un procédé de détermination d'une hauteur d'une arche dynamique de solide en vrac (3), **caractérisé en ce qu'**après l'insertion du solide en vrac (3) dans une boîte de modèle (1) orientée horizontalement avec une paroi avant (6) transparente parallèle, une paroi arrière (16) et des barrières (22) définissant une fente entre les barrières (22), où un angle d'inclinaison entre la boîte de modèle (1) et le plan horizontal est augmenté dans au moins une direction par un dispositif de positionnement jusqu'à ce qu'un mouvement uniforme du solide en vrac (3) se produise dans la boîte de modèle (1) dans la première direction, puis l'angle d'inclinaison est ensuite modifié en continu dans la première direction dans la plage de ± 5° pour créer une position stable à la fois de l'arche statique et dynamique, tandis que les valeurs géométriques de l'arche dynamique et de sa position sont déduites à l'aide d'une caméra vidéo, tandis que la mesure est répétée avec différentes valeurs de l'un des paramètres choisis suivants: diverses largeurs de la fente entre les barrières (22) dans la boîte de modèle (1), diverses teneurs en eau ajoutée au solide en vrac (3) modifiant la force de liaison entre les particules, ou l'utilisation de différentes hauteurs du solide en vrac (3) au-dessus de la fente; des valeurs mesurées de la hauteur de l'arche dynamique sont ensuite transcrites à un graphique de hauteur dynamique en fonction du paramètre choisi.

2. Le procédé de détermination d'une hauteur d'une arche dynamique de solide en vrac (3) selon la revendication 1, **caractérisé en ce que** l'inclinaison et/ou la rotation s'accompagne d'une vibration et/ou par des effets d'une force magnétique.

3. Un dispositif de test pour la détermination d'une hauteur d'une arche dynamique de solide en vrac (3) selon la revendication 1 ou 2, contenant une boîte de modèle (1) contenant une chambre de modèle (2) pour insérer le solide en vrac (3), tandis que la chambre de modèle (2) est entourée par des parois (6, 16, 26) et comprend des barrières (22) définissant une fente entre les barrières (22), où les parois sont une paroi avant (6), une paroi arrière (16), qui sont au moins partiellement transparentes et parallèles l'une à l'autre, et des parois latérales (26), et comprenant un dispositif de positionnement (4) qui est relié à la boîte de modèle (1) et qui comprend une base (12), où le dispositif de positionnement (4) est agencé pour effectuer une rotation et/ou une inclinaison de la boîte de modèle (1) autour d'au moins deux axes non parallèles; où le dispositif de test comprend en outre une caméra vidéo (7) pour l'observation du solide en vrac par la partie transparente des parois.

4. Le dispositif de test selon la revendication 3, **caractérisé en ce que** la boîte de modèle (1) est en outre complétée par des aimants permanents (29, 30), où un premier aimant permanent (29) est situé le long du bord supérieur de la boîte de modèle (1), un second aimant permanent (30) est situé le long de la paroi latérale (26).

5. Le dispositif de test selon la revendication 3 ou 4, **caractérisé en ce que** le dispositif de positionnement (4) est constitué d'une base (12), à laquelle un cadre rotatif principal (13) est réglé en rotation à l'aide d'une première broche (8) et d'un premier arbre (9) avec un premier entraînement (15), où un cadre rotatif auxiliaire (14) est réglé en rotation dans le cadre rotatif principal (13) à l'aide d'une seconde broche (18) et un second arbre (19) avec un second entraînement (25), tandis que les axes de rotation des deux arbres (9, 19) sont perpendiculaires l'un à l'autre; où la boîte de modèle (1) est reliée au cadre rotatif auxiliaire (14) et la caméra vidéo est montée sur le cadre rotatif auxiliaire (14), où la première broche (8) et le premier arbre (9) sont reliés à la base (12).

6. Le dispositif de test selon la revendication 5, **caractérisé en ce que** le cadre rotatif principal (13) est en outre relié à un premier vibreur (11) et la paroi avant (6) de la chambre de modèle (2) est équipée d'un second vibreur (21).

7. Le dispositif de test selon la revendication 3 ou 4, **caractérisé en ce que** le dispositif de positionnement (4) comprend un premier entraînement (15) relié à la base (12), un premier entraînement (15) avec un premier arbre (9) qui est en outre relié à un adaptateur (23), un deuxième entraînement (25) qui est une liaison entre l'adaptateur (23) et le second arbre (19), le second arbre (19) relié par un troisième entraînement (24) au troisième arbre (27) en outre relié à la boîte de modèle (1), tandis que le premier axe de l'arbre (9) est perpendiculaire à l'axe de l'adaptateur (23) et l'axe du second adaptateur (23) et du second arbre (19) sont identiques, tandis que l'axe du troisième arbre (27) est perpendiculaire à l'axe du deuxième arbre (19).

8. Le dispositif de test selon la revendication 3 ou 4, **caractérisé en ce que** le dispositif de positionnement (4) comprend un joint (10) pour l'inclinaison/rotation d'une boîte de modèle (1) à un angle spatial et un aimant permanent (28) pour faire tourner la boîte de modèle (1) qui est placée en regard du côté opposé du bord supérieur de la boîte de modèle (1), qui peut être déplacée dans l'espace.

9. Le dispositif de test selon la revendication 3 ou 4, **caractérisé en ce que** le dispositif de positionnement comprend un corps sphérique (31) à l'intérieur duquel se trouve la boîte de modèle (1), qui tourne autour d'un quelconque axe de rotation/inclinaison, en fonction de la direction et de l'intensité du champ magnétique entre les premiers aimants permanents (32) situés à l'intérieur du corps sphérique (31), et les seconds aimants (33) reliés à la base (12) et entourant le corps sphérique (31).

10. Le dispositif de test selon l'une quelconque des revendications de 3 à 9, **caractérisé en ce que** la boîte de modèle (1) comprend en outre un capteur de pression (5) du solide en vrac (3) situé sur les surfaces intérieures des parois latérales (26) et/ou un capteur de signal audio et/ou un capteur ultrasonore.
